# EUROPEAN PATENT APPLICATION

(11) **EP 2 781 505 A1**
(43) Date of publication of application: **24.09.2014**
(21) Application number: 14156640.6
(22) Date of filing: 02.10.2006
(51) Int. Cl.: C07C 215/40, C07C 215/42, C07C 405/00, A61K 31/5575, A61P 9/12, A61P 15/00

(54) **Positively charged water-soluble prodrugs of prostaglandins and related compounds with very high skin penetration rates**

(62) Divisional of application: 06809471.3
(71) Applicant: Techfields Biochem Co. Ltd, Shanghai N/A 200444 (CN); Yu, Chongxi, Plainfield, Illinois 60585 (US)
(72) Inventor: Yu, Chongxi, Plainfield, IL 60585 (US); Xu, Lina, Shanghai N/A 200444 (CN)
(74) Representative: Viering, Jentschura & Partner Patent- und Rechtsanwälte

(57) **Abstract**

The novel positively charged pro-drugs of prostaglandins, prostacyclins and related compounds in the general formula (2) 'Structure 2' were designed and synthesized. The compounds of the general formula (2) 'Structure 2' indicated above can be prepared from protected prostaglandins, prostacyclins, and related compounds, by reaction with suitable alcohols, thiols, or amines and coupling reagents, such as N, N'-Dicyclohexylcarbodiimide, N, N'-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris (dimethylamino)phosphonium hexafluorophosphate, et al. The positively charged amino groups of these pro-drugs not only largely increases the solubility of the drugs in water, but also bonds to the negative charge on the phosphate head group of membranes and pushes the pro-drug into the cytosol. The results suggest that the pro-drugs diffuse through human skin ∼1000 times faster than do prostaglandins, prostacyclins, and related compounds. In plasma, more than 90% of these pro-drugs can change back to the parent drugs in a few minutes. The prodrugs can be used medicinally in treating any prostaglandins, prostacyclins, and related compounds-treatable conditions in humans or animals. The prodrugs can be administered transdermally for any kind of medical treatments and avoid most of the side effects of prostaglandins, prostacyclins, and related compounds. Controlled transdermal administration systems of the prodrug enable prostaglandins, prostacyclins, and related compounds to reach constantly optimal therapeutic blood levels to increase effectiveness and reduce the side effects of prostaglandins, prostacyclins, and related compounds. Another great benefit of transdermal administration of these pro-drugs is that administering medication, especially to children, will be much easier.

## Description

### Technical Field

The present invention relates to the preparations of positively charged and water-soluble pro-drugs of prostaglandins, prostacyclins, and related compounds and their medicinal use in treating any prostaglandins, prostacyclins, and related compounds-treatable conditions in humans or animals. More specifically, the present invention is to enable quick skin penetration of prostaglandins, prostacyclins, and related compounds.

### Background Art

Natural prostaglandins and prostacyclins belong to the class of eicosanoids, a member of the group of autocoids derived from membrane phospholipids. They have been found in essentially every area of the body. The general structure of the prostaglandins is shown in Structure 1.

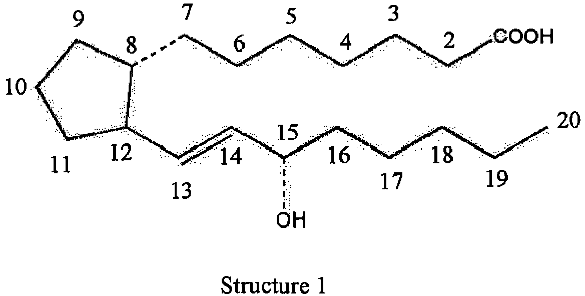

All natural prostaglandins possess a 15α-hydroxy group and a trans double bond at C-13 (William O. Foye, et al. Principles of Medicinal Chemistry, fourth edition, Williams & Wilkins, 1995, pg 538). The carboxyl-bearing chain is termed the alpha-chain and the hydroxyl-bearing chain is termed the omega-chain. The PGs are classified by the capital letters A, B, C, D, E, F, G, H, and I depending on the nature and stereochemistry of oxygen substituents at the 9- and 11- positions. The actions of the various PGs are diverse. When administered intravaginally, PGE₂ will stimulate the endometrium of the gravid uterus to contract in a manner similar to uterine contractions observed during labor. Thus, PGE₂ is therapeutically available as dinoprostone (prostin E₂, Upjohn) for use as an abortifacient. PGE₂ is also a potent stimulator of smooth muscle of the gastrointestinal (GI) tract and can elevate body temperature in addition to possessing potent vasodilating properties in most vascular tissue and also possessing constrictor effects at certain sites. PGF_{2α} shares many of PGEs' properties and is also therapeutically available as an abortifacient (Prostin F2 alpha, Upjohn). The synthetic 15-methyl derivative of PGF_{2α}, carboprost, is also therapeutically available as an abor-tifacient (Prostin 15/M, Upjohn). PGD₂ causes both vasodilation and vasoconstriction. Whereas the PGEs produce a relaxation of bronchial and tracheal smooth muscle, PGFs and PGD₂ cause contraction. PGE₁ is available as alprostadil to maintain potency of the ductus arteriosus in neonates until surgery can be performed to correct congenital heart defects. PGE₁ and its analogs may be used for the treatment of male erectile dysfunction (Yeager, James L. U.S. Pat. No. 6,693,135) and enhancing female sexual arousal (Scott, Nathan Earl, U.S. Pat. No. 6,291,528) . Prostaglandin analogs are a leading class of glaucoma drugs with a proven safety and efficacy for controlling IOP. They include bimatoprost {(Z)-7-[(1R, 2R, 3R, 5S)-3,5-Dihydroxy-2-[1E, 3S] - 3-hydroxy-5-phenyl-1-pentenyl]cyclopentyl}-5-N-ethylheptenamide}, latanoprost (13,14-dihydro-17-phenyl-18,19,20-trinor PGF_{2α} isopropyl ester), travoprost {(Z)-7-[(1 *R*, *2 R, 3 R, 5* S)-3,5-dihydroxy-2- [(1 *E*, *3 R*)-3-hydroxy-4-[(α, α, α-trifluoro-*m-*tolyl)oxy]-1-butenyl]cyclopentyl]-5-heptenoate}, and unoprostone ( 13,14-dihydro-15-keto-20-ethyl PGF_{2α}).

Unfortunately, prostaglandins, prostacyclins, and related compounds are rapidly metabolized and inactivated by various oxidative and reductive pathways. When PGs are taken orally, the first pass metabolism, which refers to the chemical breakdown of compounds in the liver and gastro-intestinal tract, can destroy and inactivate them in a few seconds . In the case of administrating PGs by injection, it is painful and in many cases requires frequent and costly office visits to treat chronic conditions, and the blood and liver can destroy and inactivate most of prostaglandins, prostacyclins, and related compounds before they reach the intended site of action.

One alternative method of administering drugs is topical delivery. Topical drug delivery has several advantages. This method helps to avoid inactivation of a drug caused by first pass metabolism in the liver and gastro-intestinal tract. It can provide local delivery of appropriate concentrations of a drug to the intended site of action without systemic exposure. Fishman (Fishman; Robert, U.S. Pat. No. 7,052,715) indicated that an additional problem associated with oral medications, is that the concentration levels which must be achieved in the bloodstream must be significant in order to effectively treat distal areas of pain or inflammation. These levels are often much higher than would be necessary if it were possible to accurately target the particular site of pain or injury. Yeager has tried to use penetration enhancer to deliver PGE₁ for the treatment of male erectile dysfunction (Yeager, James L. U.S. Pat. No. 6,693,135) . Susan Milosovich, et al. designed and prepared testosteronyl-4-dimethylaminobutyrate.HCl (TSBH), which has a lipophilic portion and a tertiary amine group that exists in the protonated form at physiological pH. They found that the prodrug (TSBH) diffuses through human skin -60 times faster than does the drug (TS) itself [Susan Milosovich, et al., J. Pharm. Sci., 82, 227(1993).

### Disclosure of Invention

### Technical Problem

The actions of the various PGs are diverse. PGs have many medicinal uses. PGE₂ and PGF_{2α} are therapeutically available. The synthetic 15-methyl derivative of PGF_{2α}, carboprost, is also therapeutically available as an abortifacient (Prostin 15/M, Upjohn). PGE₁ is available as alprostadil to maintain potency of the ductus arteriosus in neonates until surgery can be performed to correct congenital heart defects. PGE₁ and its analogs may be used for the treatment of male erectile dysfunction (Yeager, James L. U.S. Pat. No. 6,693,135) and enhancing female sexual arousal (Scott, Nathan Earl, U.S. Pat. No. 6,291,528). Prostaglandin analogs are a leading class of glaucoma drugs with a proven safety and efficacy for controlling IOP. They include bimatoprost { (Z)-7-[(1R,2R,3R,5S)-3,5-Dihydroxy-2-[1E,3S]-3-hydroxy-5-phenyl-1-pentenyl]cyclo pentyl}-5-N-ethylheptenamide}, latanoprost (13,14-dihydro-17-phenyl-18,19,20-trinor PGF_{2α} isopropyl ester), travoprost {(Z)-7-[(1*R*,2*R*,3*R*,5*S*)-3,5-dihydroxy-2-[(1*E* ,3 *R*)-3-hydroxy-4-[(α,α,α-trifluoro-*m*-tolyl)oxy]-1-butenyl]cyclopentyl]-5-heptenoate} , and unoprostone (13,14-dihydro-15-keto-20-ethyl Prostaglandin F_{2α}).

Unfortunately, prostaglandins, prostacyclins, and related compounds are rapidly metabolized and inactivated by various oxidative and reductive pathways. When PGs are taken orally, the first pass metabolism, which refers to the chemical breakdown of compounds in the liver and gastro-intestinal tract, can destroy and inactivate them in a few seconds. In the case of injection, administration of PGs is painful and in many cases requires frequent and costly office visits to treat chronic conditions and the blood and liver can destroy and inactivate most of the prostaglandins, prostacyclins, and related compounds before they reach the intended site of action . When PGs is topically applied to eyes for treating glaucoma and ocular hypertension, they may cause blurred vision, inflamed or infected eyes or eyelids, burning, stinging, or discomfort due to their slow penetration through the eye membrane.

### Technical Solution

This invention relates to the preparation of novel positively charged pro-drugs of prostaglandins, prostacyclins, and related compounds and their medicinal use. The pro-drugs of prostaglandins, prostacyclins, and related compounds have the general formula (2) 'Structure 2'.

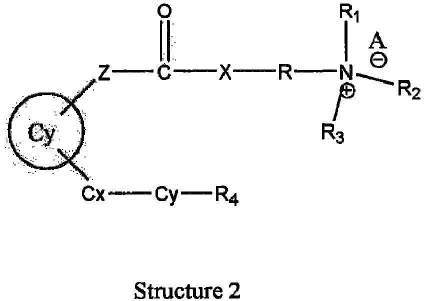

Wherein, R₁ represents H, one of any alkyl, alkyloxyl, alkenyl or alkynyl residues having 1 to 12 carbon atoms, aryl or heteroaryl residues; R represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; X represents O, S, or NH; A⁻ represents Cl⁻, Br⁻, F⁻, I⁻, AcO⁻, citrate, or any negative ions; R represents a branched or straight chain, -(CH₂)ₙ-, wherein n=0, 1, 2, 3, 4, 5, 6, 7, 8, 9, 10 ......, an aryl or heteroaryl group; Z represents -(CH₂)₆-, -(CH₂)ₙ-, -(CH₂)ₘ-O-CH₂-, -(CH₂)ₘ-S-CH₂-, -CH₂C≡C-(CH₂)ₙ-, -CH₂C≡C-(CH₂)ₙ-O-CH₂-, -CH₂C≡C-(CH₂)ₙ-S-CH₂-, -CH₂ -CO-(CH₂)ₙ-, -CH₂-CH=C=CH-(CH₂)ₙ-, -CH₂-CH=C=CH-O-(CH₂)ₙ-, -CH₂-CH=C=CH-S-(CH₂)ₙ-,

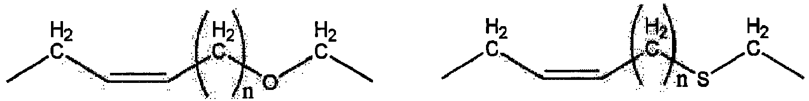

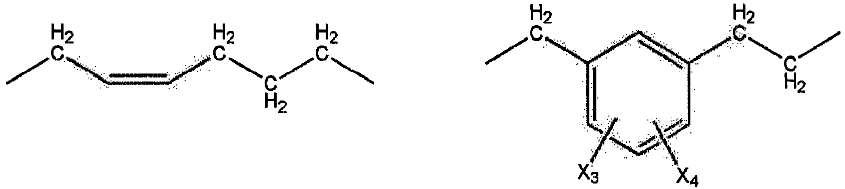

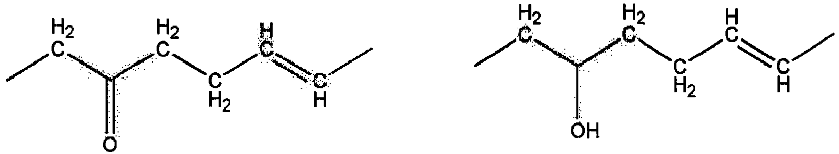

Wherein X₃ and X₄ represent H, OH, Cl, F, OCH₃, S-CH₃, CH₃, C₂H₅, CH=CH2, CH₂ CH=CH₂, and CF₃; m and n have a value of 0 to 8 inclusive; Cx-Cy is -CH₂-CH₂-, S-CH₂-, -O-CH₂-, -C≡C-, or -CH=CH-; R₄ represents:

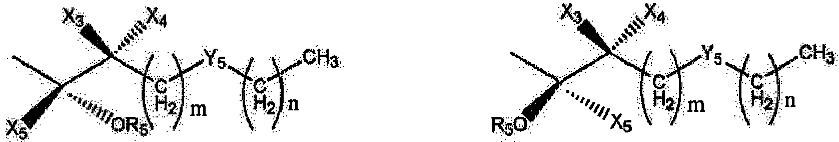

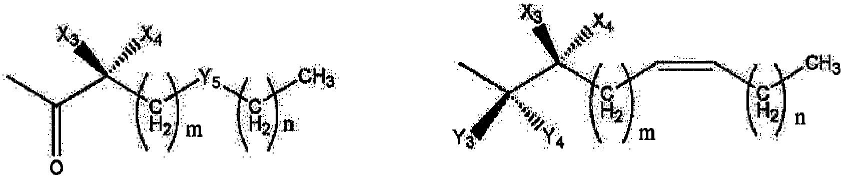

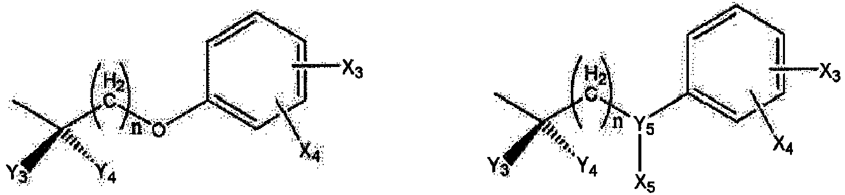

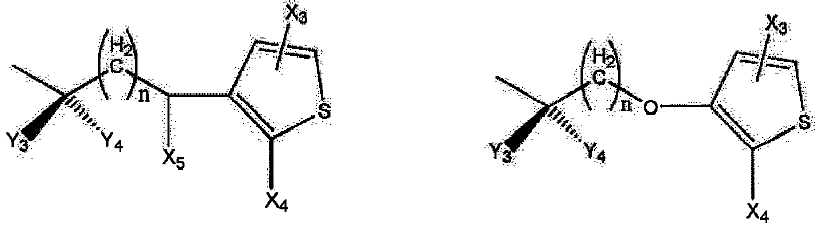

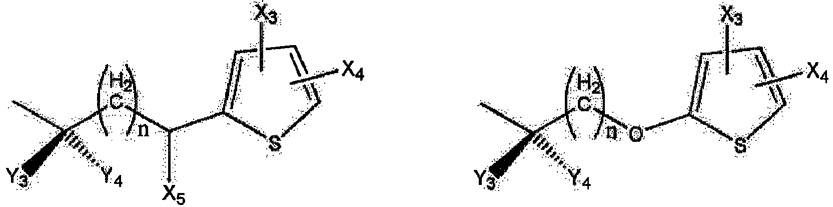

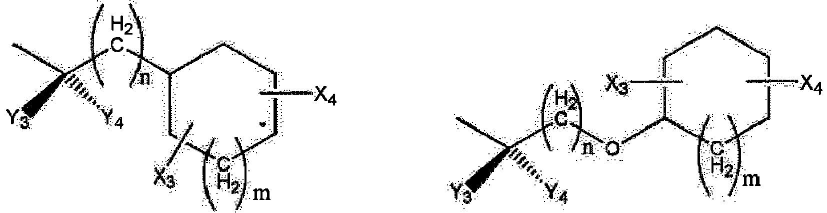

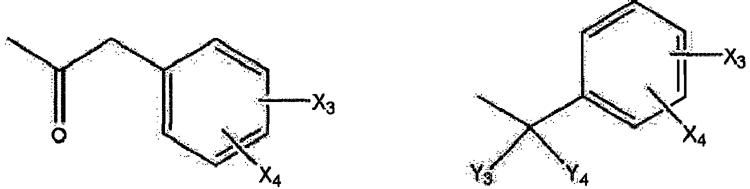

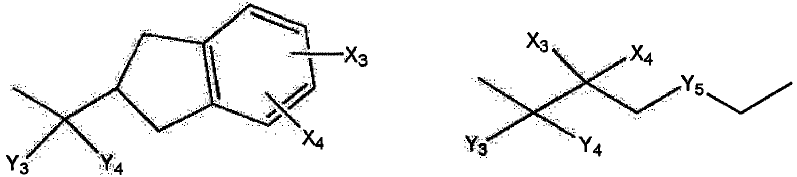

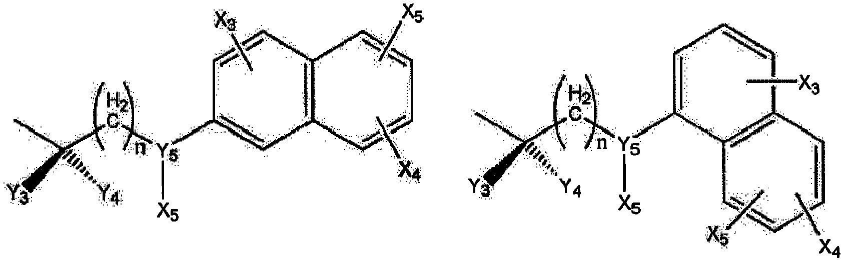

Wherein R₅ represents H, OH, acetyl, propionyl, isobutyryl, butyryl, pivaloyl, valeryl, and isovaleryl; X₃, X₄, and X₅ represent H, OH, Cl, F, OCH₃, S-CH₃, CH₃, C₂H ₅, CH=CH2, CH₂CH=CH₂, and CF₃; Y₃ and Y₄ taken alone are different and are H, OH, OR₅, OOH, OCOCH₃, OCOC₂H₅, OCOC₃H₇, OCOC₄H₉, OCOC₅H₁₁, OCOC₆H₁₃, CH₃, CH₂OH, CH₂OCOCH₃, CH₂OCOC₂H₅, CH₂OCOC₃H₇, CH₂OCOC₄H₉, Cl, F, Br, I, or taken together are oxygen or 2 hydrogens. Y₅ is CH₂, NH, S, or O, m and n have a value of 0 to 6 inclusive. 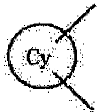 represents :

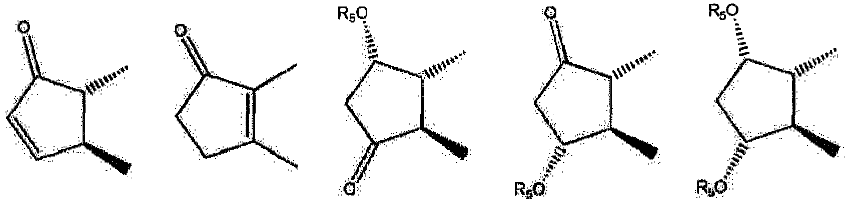

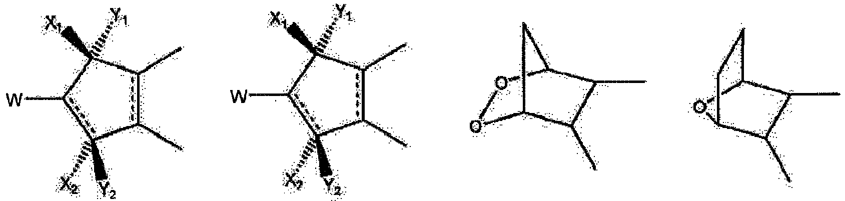

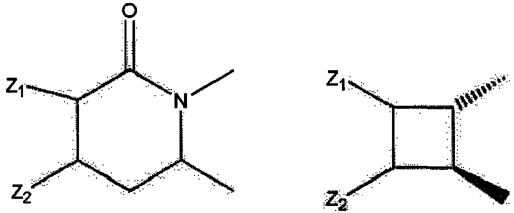

Wherein R₅ represents H, OH, acetyl, propionyl, isobutyryl, butyryl, pivaloyl, valeryl, and isovaleryl; X₁ and Y₁ taken alone are different and are H, OH, OR₅, OOH, OCOCH ₃, OCOC₂H₅, OCOC₃H₇, OCOC₄H₉, OCOC₅H₁₁, OCOC₆H₁₃, CH₂-OH, Cl, F, Br, I, or taken together are oxygen or 2 hydrogens; X₂ and Y₂ taken alone are different and are H, OH, OOH, OCOCH₃, OCOC₂H₅, OCOC₃H₇, OCOC₄H₉, OCOC₅H₁₁, OCOC₆H₁₃, CH₂-OH, Cl, F, Br, I, nothing (when the dashed bond is a double bond) or taken together are oxygen or 2 hydrogens; Z₁ and Z₂ represent H, OH, OR₅, OOH, OCOCH₃, OCOC₂H₅*,* OCOC₃H₇, OCOC₄H₉, OCOC₅H₁₁, OCOC₆H₁₃, CH₂-OH, or Cl. W represents H, CH₃, Cl, F, Br, I, or OH; the dashed bonds represent a single or double bond; All R, -(CH₂)ₙ- or -(CH₂)ₘ-groups are branched or straight chains and may include C, H, O, S, or N atoms and may have single, double, and triple bonds. Any CH ₂ groups may be replaced with O, S, or NH.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. Membranes are bilayers, with the hydrophilic head groups facing outward into the aqueous regions on either side. Very hydrophilic drugs cannot pass the hydrophobic layer of a membrane and very hydrophobic drugs will stay in the hydrophobic layer as part of the membrane due to their similarities and cannot efficiently enter the cytosol on the inside.

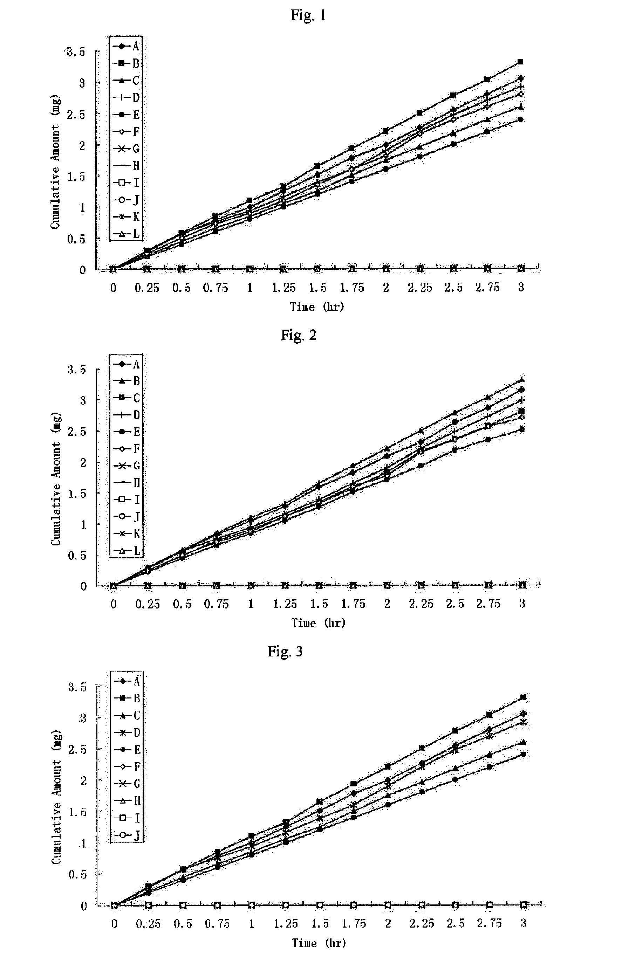

The goal of this invention is to make prostaglandins, prostacyclins, and related compounds administrable transdermally (topical application) by increasing their solubility in the moisture available on the skin surface and their penetration rate through the membrane and skin barrier. These novel pro-drugs of prostaglandins, prostacyclins, and related compounds have two structural features in common: they have a lipophilic portion and a primary, secondary, or tertiary amine group that exists in the protonated form (hydrophilic part) at physiological pH. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water. The solubility of theses pro-drugs of prostaglandins, prostacyclins, and related compounds is >100 mg/ml and the solubility of prostaglandins, prostacyclins, and related compounds is <0.01 mg/ml. In many instances, the lowest or rate-limiting step in the sequence is the dissolution of the drug. Prostaglandins, prostacyclins, and related compounds have a very low solubility in the moisture available on the skin surface, and they will not pass across the barrier of skin in a molecular form. They stay on the outside of the membranes of the eye or skin for a long time and thus, may cause pain, itching, or swelling of the eye or skin. When these new pro-drugs are administered transdermally in a dosage form such as a solution, spray, lotion, ointment, emulsion or gel, they will dissolve in the moisture available on the eye or skin surface immediately. The positive charge on the amino groups of these pro-drugs will bond to the negative charge on the phosphate head group of a membrane. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol, a semi-liquid concentrated aqueous solution or suspension. Due to the short stay outside of the membranes of the eye or skin, the pro-drugs will not cause burning, pain, itching, or swelling of the eye or skin. The penetration rates of these prodrugs through human skin were measured in vitro by using modified Franz cells, which were isolated from human skin tissue (360-400 µm thick) of the anterior and posterior thigh areas. The receiving fluid consisted of 2 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 10% solution of some of the prodrugs or a 10% suspension of some of prostaglandins, prostacyclins, and related compounds in 0.2mL of pH 7.4-phosphate buffer (0.2M) are shown in Figure 1, Figure 2, and Figure 3. Apparent flux values of 1.01 mg, 1.10 mg, 0.85 mg, 0.94 mg, 0.80 mg, 0.90 mg, 1.05 mg, 1.09 mg, 0.91 mg, 0.95 mg, 0.85 mg, 0.88 mg, 1.01 mg, 1.11 mg, 0.86 mg, 0.92 mg, 0.81 mg, 0.001 mg, 0.001 mg, 0.001 mg, 0.001 mg, 0.001 mg, 0.001 mg, 0.001 mg, 0.001 mg 0.001 mg, 0.001 mg, 0.001 mg, 0.001 mg, 0.001 mg, 0.001 mg, 0.001 mg, 0.001 mg, and 0.001 mg/cm²/h were calculated for N,N-diethylaminoethyl
11,15-dihydroxy-9-oxoprost-13-en-1-oate.AcOH, N,N-diethylaminoethyl
11,15-dihydroxy-9-oxoprosta-5,13-dien-1-oate.AcOH, N,N-diethylaminoethyl
9,11,15-trihydroxyprost-13-en-1-oate.AcOH, N,N-diethylaminoethyl
9,11,15-trihydroxyprosta-5,13-dien-1-oate.AcOH, N,N-diethylaminoethyl
9,11,15-trihydroxy-15-methylprosta-5,13-dien-1-oate.AcOH, N,N-diethylaminoethyl
9,11,15-trihydroxy-15-methylprosta-4,5,13-trien-1-oate.AcOH, N,N-diethylaminoethyl
9,11-dihydroxy-15-keto-20-ethylprost-5,13-dien-1-oate.AcOH
(9,11-dihydroxy-15-keto-20-ethylprostaglandin F_{2α} N,N-diethylaminoethyl ester),
N,N-diethylaminoethyl 11,16-dihydroxy-9-oxo-16-methylprost-13-en-1-oate.AcOH,
N,N-diethylaminoethyl (Z)-7-{(1*R*,2*R*,3*R*,5*S*)-3,5-dihydroxy-2-[(1*E*,3*R* )-3-hydroxy-4-[(α,α,α-trifluoro-*m*-tolyl)oxy]-1-butenyl]cyclopentyl}-5-heptenoate .AcOH, N,N-diethylaminoethyl (Z)-7{(1R, 2R, 3R, 5S)3,
5-dihydroxy-2-[(3R)-3-hydroxy-5-phenylpentyl]cyclopentyl}-5-heptenoate.AcOH, N,N-diethylaminoethyl (Z)-7-{(1R,2R,3R,5S)-3,5-Dihydroxy-2-[( 1E,3S)-3-hydroxy-5-phenyl-1-pentenyl]cyclopentyl}-hepteno ate.AcOH, N,N-diethylaminoethyl
11,15-dihydroxy-16,16-dimethyl-9-oxoprosta-2,13-dien-1-oate.AcOH, N,N-diethylaminoethyl
7-[3-hydroxy-2-(3-hydroxy-4-phenoxy-1-butenyl)-5-oxocyclopentyl]-5-heptenoate.Ac OH, N,N-diethylaminoethyl
6,9-epoxy-11,15-dihydroxyprosta-5,13-dien-1-oate.AcOH, N,N-diethylaminoethyl
7-{3,5-dihydroxy-2-[3-hydroxy-4-(3-trifluoromethylphenoxy)-1-butenyl]cyclopentyl}-5-heptenoate.AcOH, N,N-diethylaminoethyl
7-{2-[4-(3-chlorophenoxy)-3-hydroxy-1-butenyl]-3,5-dihydroxycyclopentyl}-5-hepten oate.AcOH, N,N-diethylaminoethyl
7-[3,5-dihydroxy-2-(3-hydroxy-4-phenoxy-1-butenyl)cyclopentyl]-4,5-heptadien-1-oat e.AcOH, PGE₁, PGE₂, PGF_{1α}, PGF_{2α}, carboprost, prostalene, unoprostone, misoprostol, travoprost , latanoprost, bimatoprost , gemeprost, sulprostone, PGI₂, fluprostenol, cloprostenol, and fenprostalene respectively diffusing through human skin. The results suggest that the pro-drugs diffuse through human skin -1000 times faster than do prostaglandins, prostacyclins, and related compounds. The results suggest that the positive charge on the dialkyaminoethyl group has a very important role in the passage of the drug across the membrane and skin barrier. Other prodrugs of the general formula (2) 'Structure 2' have very high penetration rates and are very close to that of N,N-diethylaminoethyl 11,15-dihydroxy-9-oxoprost-13-en-1-oate.AcOH.

Prostagalndins are highly effective ocular hypotensive agents and are ideally suited for the long-term medical management of glaucoma (Woodward, D.F. et al., U.S. Pat. No.5,688,819). Such prostaglandins include PGA, PGB, PGD, PGF_{2α,} PGF_{1α}**,** PGE₂, PGE₁ and their alkyl esters were reported to possess ocular hypotensive activity, but generally cause inflammation, as well as surface irritation characterized by conjunctival hyperemia and edema. Certain phenyl and phenoxy mono, tri and tetra nor prostaglandind and their esters disclosed in European Patent Application 0,364,417 are useful in the treatment of glaucoma or ocular hypertension. Buchmann et al. (Buchmann. et al., U.S. Pat. No. 5,756,818) disclosed certain species of cyclopentane heptanoic acid, 2-cycloalkyl, or aryalkyl compounds said to be suitable for lowering intraocular pressure. Woodward et al. (Woodward, D.F. et al., U.S. Pat. No. 5688819) disclosed that cyclopentane heptanoic acid, 2-cycloalkyl, or aryalkyl compounds are useful in the treatment of glaucoma or ocular hypertension. Recently, unoprostone , travoprost , latanoprost, and bimatoprost are becoming a leading class of glaucoma drugs. These drugs all have side effects due to their very low penetration rates. These side effects included blurred vision, redness, a sensation of a foreign body, discoloration of the iris, itching, burning, stinging, dryness of the eyes, increased tearing, eye pain and other eye-related discomfort.

The ability of certain compounds of the present invention to reduce intraocular pressure (IOP) was evaluated in cats with ocular hypertension produced by previously done laser trabeculoplastry. IOP was determined with a pneumatonometer after light corneal anesthesia with dilute proparacaine. Baseline IOP (in mm Hg) was determined prior to treatment with the test compound aqueous solution. 6 divided doses were administered over a period of 3 days (once every 12 hours). IOP was determined 24 hours after the initial dose and then once every 12 hours. The therapeutically efficient amount is typically between 0.001 and 0.01% in pH 7.2 phosphate buffer (0.1 M). The treatment was carried out in that one drop (about 30 micro liter) of the composition . The results of N,N-diethylaminoethyl 11,15-dihydroxy-9-oxoprost-13-en-1-oate.AcOH (A), N,N-diethylaminoethyl 11,15-dihydroxyl-9-oxoprosta-5,13-dien-1-oate.AcOH (B), N,N-diethylaminoethyl-9,11,15-trihydroxyprosta-5,13-dien-1-oate.AcOH (C), N,N-diethylaminoethyl 9,11-dihydroxy-15-keto-20-ethylprost-5,13-dien-1-oate.AcOH (9,11-dihydroxy-15-keto-20-ethylprostaglandin F_{2α} N,N-diethylaminoethyl ester) (D), N,N-diethylaminoethyl (Z)-7- {(1*R*,2*R*,3*R*,5*S*)-3,5-dihydroxy-2-[(1*E*,3*R* )-3-hydroxy-4-[(α,α,α-trifluoro-*m*-tolyl)oxy]-1-butenyl]cyclopentyl}-5-heptenoate .AcOH (E), N,N-diethylaminoethyl (Z)-7{(1R, 2R, 3R, 5S) - 3, 5-dihydroxy-2-[(3R)-3-hydroxy-5-phenylpentyl]cyclopentyl}-5-heptenoate.AcOH (F), and N,N-diethylaminoethyl (Z)-7-{(1R,2R,3R,5S)-3,5-Dihydroxy-2-[( 1E,3S)-3-hydroxy-5-phenyl-1-pentenyl]cyclopentyl}-hepteno ate.AcOH (G) are shown in table 1.

**Table 1: Intraocular pressure redution by the prodrugs of natural prostaglandin and modified analogs as determined in cat.**

| Compound | Baseline | Dose% | Time after administration (hours) | | | | |
|---|---|---|---|---|---|---|---|
| | | | 24 | 36 | 48 | 60 | 72 |
| A | 22.1±0.7 | 0.001 | 15.3±0.4 | 15.1±0.5 | 15.0±0.4 | 15.1±0.5 | 15.4±0.4 |
| B | 22.3±0.6 | 0.001 | 15.7±0.5 | 15.8±0.5 | 15.9±0.4 | 15.7±0.4 | 15.9±0.5 |
| C | 22.5±0.7 | 0.001 | 15.5±0.5 | 15.6±0.6 | 15.7±0.5 | 15.6±0.7 | 15.5±0.6 |
| D | 22.0±0.6 | 0.01 | 16.5±0.6 | 16.7±0.4 | 16.8±0.6 | 17.1±0.5 | 16.8±0.5 |
| E | 22.1±0.4 | 0.001 | 15.1±0.4 | 15.0±0.5 | 15.0±0.4 | 149±0.3 | 15.1±0.4 |
| F | 22.5±0.5 | 0.001 | 15.6±0.5 | 15.7±0.5 | 15.6±0.5 | 15.5±0.4 | 15.6±0.6 |
| G | 22.4±0.6 | 0.001 | 15.2±0.6 | 15.4±0.5 | 15.5±0.4 | 15.4±0.6 | 15.4±0.5 |

Irritative effect or ocular discomfort in the cat eye of naturally occurring and modified prostaglandins and their novel prodrugs were evaluated during the first hours after the topical application of the respective test drug. The ocular discomfort was graded on a scale from 0 to 4, 0 indicating complete absence of any signs of discomfort, and 4 indicating maximal irritation as obvious from complete lid closure. The results are shown in table 2.

**Table 2: Irritative effect of naturally occurring and modified prostaglandins and their novel prodrugs during the first 2 hours after topical application of the respective test drug**

| Compound | Dose % | Degree of Irritative Effect |
|---|---|---|
| PGE₁ | 0.001 | 4 |
| A | 0.001 | 1 |
| PGE₂ | 0.001 | 3.5 |
| B | 0.001 | 1 |
| PGF_{2α} | 0.001 | 3.5 |
| C | 0.001 | 1 |
| Unoprostone | 0.01 | 2.5 |
| D | 0.01 | 1 |
| Travoprost | 0.001 | 2.5 |
| E | 0.001 | 1 |
| Latanoprost | 0.001 | 2.5 |
| F | 0.001 | 1 |
| Bimatoprost | 0.001 | 2.5 |
| G | 0.001 | 1 |

Conjunctival hyperemia in the rabbit eye of naturally occurring and modified prostaglandins and their novel prodrugs was evaluated during the first 2 hours after topical application of the respective test drug. The conjunctival hyperemia was graded on a scale from 0 to 4, 0 indicating complete absence of any hyperemia, and 4 indicating marked hyperemia with conjunctive chemosis. The results are shown in table 3.

**Table 3: Conjunctival hyperemia in the rabbit eye of naturally occurring and modified prostaglandins and their novel prodrugs during the first 2 hours after the topical application of the respective test drug.**

| Compound | Dose % | Degree of Irritative Effect |
|---|---|---|
| PGE₁ | 0.001 | 4 |
| A | 0.001 | 1 |
| PGE₂ | 0.001 | 4 |
| B | 0.001 | 1 |
| PGF_{2α} | 0.001 | 4 |
| C | 0.001 | 1 |
| Unoprostone | 0.01 | 2.5 |
| D | 0.01 | 1 |
| Travoprost | 0.001 | 2.5 |
| E | 0.001 | 1 |
| Latanoprost | 0.001 | 2.5 |
| F | 0.001 | 1 |
| Bimatoprost | 0.001 | 2.5 |
| G | 0.001 | 1 |

The results show that these prodrugs are superior to their parent drugs for the treatment of ocular hypertension and glaucoma. They exhibit excellent intraocular pressure lowering effects, and cause no side effects or very mild side effects. Prostaglandins and related compounds are very lipophilic. When PGs are topically applied to eyes, they do not dissolve in the aqueous humor of the eye. They stay outside of the eye membranes for a long time and thus, may cause pain, itching, or swelling of the eye. When prostaglandins enter eye membranes, they will stay in the hydrophobic layer as part of the membrane due to their similarities and cannot efficiently enter the cytosol on the inside. When the prodrugs of prostaglandins are topically applied to eyes, they will dissolve in the aqueous humor of eye immediately. The positive charge on the amino groups of these pro-drugs will bond to the negative charge on the phosphate head group of membrane of eye. Thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol. Due to the short stay outside of the membranes of the eye or skin and thus, the pro-drugs will not cause burning, pain, itching, or swelling of the eye.

Prostaglandins can be used for the treatment of male erectile dysfunction (Yeager; J.L., et al. U.S. Pat. No. 6,693,135) and enhancing female sexual arousal (Scott, N. E. U.S. Pat. No. 6291528). However, working alone, prostaglandins formulations do not sufficiently permeate the skin to provide drug concentration levels . In one commercially available form (MUSE.RTM, Vivus, Menlo Park Calif.), alprostadil (PGE₁) is administered in a pellet deposited in the urethra using an applicator with a hollow stem 3.2 cm in length and 3.5 mm in diameter (Padma-Nathan, H., et al., N. Engl. J. Med., 336: 1-7 (1997) for treatment of impotence. The side effects of this treatment are penile pain and minor urethral trauma. Transurethral delivery of PGE₁ or PGE₂ is a highly effective means of treating impotence, but there are undesirable side effects that include a sensation of urethral burning or pain and cavernous sinus aching in the genital area. Transurethral delivery of PGs has another problem with removal to terminate delivery of the composition and can result in overdosing as well as the delivery of excess PGs to the vagina of the partner.

In one commercially available form, PGE₁ is administered by intracavernosal injection. The principal side effect of intracavernosal injection of alprostadil (PGE₁) is pain, an incidence of fibrosis and scar formation at the site of injection.

The novel prodrugs in the invention can diffuse through human skin at a very high rate (∼1 mg/h/cm²) and can provide almost side-effects-free methods of treating erectile dysfunction or enhancing female sexual arousal. About 0.01 ml of 0.0005%[∼0.05µg (microgram)] N,N-diethylaminoethyl 11,15-dihydroxy-9-oxoprost-13-en-1-oate.AcOH in pH 7.0 phosphate buffer (0.1 M) was applied to genital area of male rats (30 rats) once perday for 5 days. The results showed a 6 fold increase in solicitation and a 4 fold increase in copulation in rats that were given the drug compared to those that were not given the drug.

Then same amount of N,N-diethylaminoethyl 11,15-dihydroxy-9-oxoprost-13-en-1-oate.AcOH in pH 7.0 phosphate buffer (0.1 M) was applied to genital area of both male rats (30 rats) and female rats (30 rates) once perday for 5 days. The results showed a 6 fold increase in solicitation and a 6 fold increase in copulation in rats that were given the drug compared to those that were not given the drug. The most important thing is that rats that were given the drug did not show any discomfort.

Prostaglandins selected from the group consisting of natural and synthetic analogs of the PGE, PGA, and PGF are useful for reducing systemic blood pressure. 0.02 mg of N,N-diethylaminoethyl 11,15-dihydroxy-9-oxoprosta-5,13-dien-1-oate.AcOH (A) and N,N-diethylaminoethyl 11,16-dihydroxy-9-oxo-16-vinylprosta-5,13-dien-1-oate.AcOH (B) in 0.3 ml of pH 7.0 phosphate buffer (0.1 M) were applied to the back of spontaneously hypertensive rats (after intake of the tungsten-enriched diet. The blood pressure was recorded continuosly on a multichannel physiograph. The results are shown in table 4.

**Table 4: Effect of the prodrugs of prostaglandins on the mean arterial blood pressure in spontaneously hypertensive rats. All values are reported as mean± SD.**

| Compound | Baseline | Time after administration (hours) | | | | |
|---|---|---|---|---|---|---|
| | | 1 | 2 | 3 | 4 | 5 |
| A | 181.4±7.5 | 148.2±7.4 | 143.2±7.6 | 142.2±6.4 | 143.2±7.8 | 145.2±7.0 |
| B | 183.5±8.6 | 155.2±8.2 | 153.2±6.8 | 150.2±7.8 | 153.2±7.0 | 155.2±6.4 |

The mean arterial blood pressure in spontaneously hypertensive rats was significantly reduced after the transdermal administration of the prodrugs of prostaglandins and rats that were given the drug did not show any discomfort

The compounds of the general formula (2) 'Structure 2' indicated above can be prepared from protected prostaglandins, prostacyclins, and related compounds , by reaction with compounds of the general formula (3) 'Structure 3' by using coupling reagents, such as N,N'-Dicyclohexylcarbodiimide, N, N-Diisopropylcarbodiimide, O-(Benzotriazol-1-yl)-N,N,N',N'-tetramethyluronium tetrafluoroborate, O-(Benzotriazol-1-yl)-N,N,N',N'-tetramethyluronium hexafluorophosphate, Benzotriazol-1-yl-oxy-tris(dimethylamino)phosphonium hexafluorophosphate, et al.

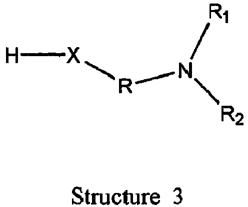

Wherein, R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R represents a branched or straight chain, -(CH₂)ₙ-, wherein n=0, 1, 2, 3, 4, 5, 6, 7, 8, 9, 10 ......, an aryl or heteroaryl group; X represents O, S or NH; and n=0, 1, 2, 3, 4, 5, 6, 7, 8, 9, 10......

When X represents O, the compounds of the general formula (2) 'Structure 2' indicated above can be prepared from metal salts, organic base salts , or immobilized base salts of prostaglandins, prostacyclins and related compounds, by reaction with compounds of the general formula (4) 'Structure 4'.

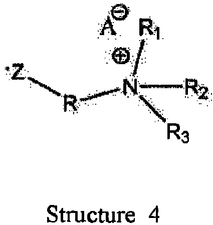

Wherein, R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; R represents a branched or straight chain, -(CH₂)ₙ-, wherein n=0, 1, 2, 3, 4, 5, 6, 7, 8, 9, 10 ......, an aryl or heteroaryl group; Z represents halogen, or p-toluenesulphonyl, A⁻ represents Cl⁻, Br⁻, F, I⁻, AcO⁻, citrate, or any negative ions.

### Advantageous Effects

These pro-drugs of prostaglandins, prostacyclins and related compounds in the present invention have a lipophilic portion and a hydrophilic portion (the amine groups that exist in the protonated form at physiological pH). The positively charged amino groups of these pro-drugs have two major advantages. First, it largely increases the solubility of the drugs in water; when these new pro-drugs are administered transdermally in a dosage form such as a solution, spray, lotion, ointment, emulsion or gel, they will mix with moisture on the skin, eye, genital area, mouth, nose, or other part of the body immediately. Second, the positive charge on the amino group of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay on the skin, eye, genital area, mouth, nose, or other part of the body, the pro-drugs will not cause itching, burning or pain. Experiment results show that more than 90% of the pro-drugs were changed back to the parent drugs in a few minutes. The pro-drugs have a much better absorption rate and as transdermal administration avoids the first pass metabolism, the pro-drugs will be stronger than prostaglandins, prostacyclins and related compounds at the same dosage. Another great benefit of transdermal administration of these pro-drugs is that administering medication, especially to children, will be much easier.

### Description of Drawings

Figure 1: Cumulative amounts of N,N-diethylaminoethyl 11,15-dihydroxy-9-oxoprost-13-en-1-oate.AcOH (A, 10% solution), N,N-diethylaminoethyl 11,15-dihydroxyl-9-oxoprosta-5,13-dien-1-oate.AcOH (B, 10% solution), N,N-diethylaminoethyl 9,11,15-trihydroxyprost-13-en-1-oate.AcOH (C, 10% solution), N,N-diethylaminoethyl 9,11,15-trihydroxyprosta-5,13-dien-1-oate.AcOH (D, 10% solution), N,N-diethylaminoethyl 9,11,15-trihydroxy-15-methylprosta-5,13-dien-1-oate.AcOH (E, 10% solution), N,N-diethylaminoethyl 9,11,15-trihydroxy-15-methylprosta-4,5,13-trien-1-oate.AcOH (F, 10% solution), PGE ₁(G, 10% suspension), PGE₂ (H, 10% suspension), PGF_{1α} (I, 10% suspension), PGF_{2α} (J, 10% suspension), carboprost (K, 10% suspension), prostalene (L, 10% suspension), crossing isolated human skin tissue in Franz cells (n=5). In each case, the vehicle was a pH 7.4 phosphate buffer (0.2 M).
Figure 2: Cumulative amounts of N,N-diethylaminoethyl 9,11-dihydroxy-15-keto-20-ethylprost-5,13-dien-1-oate.AcOH (A, 10% solution), N,N-diethylaminoethyl 11,16-dihydroxy-9-oxo-16-methylprost-13-en-1-oate.AcOH (B, 10% solution), N,N-diethylaminoethyl (Z)-7- {(1*R* ,2*R* ,3*R* ,5*S* )-3,5-dihydroxy-2-[(1*E*,3*R*)-3-hydroxy-4-[(α,α,α-trifluoro-*m*-tolyl)oxy]-1- butenyl] cyclopentyl}-5-heptenoate .AcOH (C, 10% solution), N,N-diethylaminoethyl (Z)-7{(1R, 2R, 3R, 5S)3, 5-dihydroxy-2-[(3R)-3-hydroxy-5-phenylpentyl] cyclopentyl}-5-heptenoate .AcOH (D, 10% solution), N,N-diethylaminoethyl (Z)-7-{(1R,2R,3R,5S)-3,5-Dihydroxy-2-[(1E,3S)-3-hydroxy-5-phenyl-1-pentenyl]cyclopentyl}-hepteno ate.AcOH (E, 10% solution), N,N-diethylaminoethyl 11,15-dihydroxy-16,16-dimethyl-9-oxoprosta 2,13-dien-1-oate.AcOH (F, 10% solution), unoprostone (G, 10% suspension), misoprostol (H, 10% suspension), travoprost (I, 10% suspension), latanoprost (J, 10% suspension), bimatoprost (K, 10% suspension), gemeprost, (L, 10% suspension), crossing isolated human skin tissue in Franz cells (n=5). In each case, the vehicle was a pH 7.4 phosphate buffer (0.2 M).
Figure 3: Cumulative amounts of N,N-diethylaminoethyl 7-[3-hydroxy-2-(3-hydroxy-4-phenoxy-1-butenyl)-5-oxocyclopentyl]-5-heptenoate.Ac OH (A, 10% solution), N,N-diethylaminoethyl 6,9-epoxy-11,15-dihydroxyprosta-5,13-dien-1-oate.AcOH (B, 10% solution), N,N-diethylaminoethyl 7-{3,5-dihydro xy-2-[3-hydroxy-4-(3-trifluoromethylphenoxy)-1-butenyl]cyclopentyl}-5-heptenoate.AcO H (C, 10% solution), N,N-diethylaminoethyl 7-{2-[4-(3-chlorophenoxy)-3-hydroxy-1-butenyl]-3,5-dihydroxycyclopentyl}-5-hepten oate.AcOH (D, 10% solution), N,N-diethylaminoethyl 7-[3,5-dihydroxy-2-(3-hydroxy-4-phenoxy-1-butenyl)cyclopentyl]-4,5-heptadien-1-oat e.AcOH (E, 10% solution), sulprostone (F, 10% suspension), PGI₂ (G, 10% suspension), fluprostenol (H, 10% suspension), cloprostenol (I, 10% suspension), and fenprostalene (J, 10% suspension), crossing isolated human skin tissue in Franz cells (n=5). In each case, the vehicle was a pH 7.4 phosphate buffer (0.2 M).

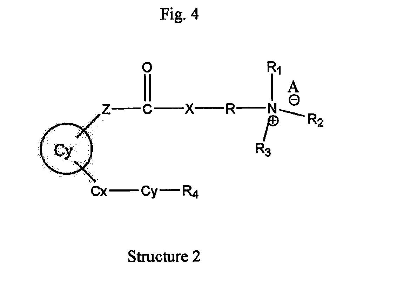

Figure 4. Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; X represents O, S, or NH; A⁻ represents Cl⁻, Br⁻, F, I⁻, AcO⁻, citrate, or any negative ions; R represents a branched or straight chain, -(CH₂)ₙ-, wherein n=0, 1, 2, 3, 4, 5, 6, 7, 8, 9, 10 ......, an aryl or heteroaryl group; Z represents the alpha-chain and Cₓ-C_{y}-R₄ represents the omega-chain. Cy represents the cyclopentyl system of prostaglandins.

### Best Mode

### Preparation of N,N-diethylaminoethyl 11,15-dihydroxy-9-oxoprost-13-en-1-oate.AcOH

37.7 g (0.1 mol) of sodium 11,15-dihydroxy-9-oxoprost-13-en-1-oate was dissolved in 100 ml of acetonitrile. 26.1 g (0.1 mol) of 2-Bromo-N,N-diethylethylamine.HBr and 8.6 g of sodium bicarbonate were added into the reaction mixture. The mixture was stirred for overnight at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 42 g of the desired product (81.8%). Hygroscopic product; Solubility in water: 100 mg/ml; Elementary analysis: C₂₈H₅₁NO₇; MW: 513.37. Calculated % C: 65.47; H: 10.01; N: 2.73; O: 21.80; Found % C: 65.42; H: 10.03; N: 2.70; O: 21.85. ¹H-NMR (400 MHz, D₂O): δ: 0.96 (t, 3H), 1.25-1.33 (m, 12H), 1.48-1.53 (m, 4H) 1.55 (t, 6H), 1.68 (m, 2H), 2.08 (m, 1H), 2.18 (s, 3H), 2.21 (m, 2H), 2.25 (t, 2H), 2.77 (m, 1H), 3.22 (m, 4H), 3.50 (m, 2H), 3.76 (m, 1H), 3.90 (m, 1H), 4.52 (m, 2H), 5.65-5.69 (m, 2H).

### Mode for Invention

### Preparation of N,N-diethylaminoethyl 11,15-diacetoxy-9-oxoprosta-5,13-dien-1-oamide.AcOH.

43.7 g (0.1 mol) of 11,15-diacetoxy-9-oxoprosta-5,13-dien-1-oic acid was dissolved in 300 ml of chloroform. 20.6 g of N, N-Dicyclohexylcarbodiimide was added into the reaction mixture. 11.7 g of N,N-diethylaminoethylamine was added into the reaction mixture. The mixture was stirred for 3 hours at RT. The solid was removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 45 g of the desired product (85.8%). Hygroscopic product; Solubility in water: 100 mg/ml; Elementary analysis: C₃₄H₅₉NO₉S; MW: 657.90. Calculated % C: 62.07; H: 9.04; N: 2.13; O: 21.89; S: 4.87; Found % C: 62.02; H: 9.06; N: 2.11, O: 21.95; S: 4.86. ¹H-NMR (400 MHz, D₂O): δ: 0.95 (t, 3H), 1.25-1.33 (m, 14H), 1.54 (m, 2H) 1.56 (t, 6H), 1.62 (m, 2H), 1.99 (m, 2H), 2.01 (s, 3H), 2.02 (s, 3H), 2.05 (s, 3H), 2.10 (m, 1H), 2.18 (s, 3H), 2.35 (t, 2H), 2.77 (m, 1H), 3.22 (m, 4H), 3.35 (m, 2H), 3.89 (m, 2H), 3.97 (m, 1H), 4.02 (m, 1H), 4.60 (m, 1H), 5.45-5.69 (m, 2H).

### Preparation of S-(N,N-dimethylaminoethyl) 9,11,15-triacetoxythioprost-13-en-1-oate.AcOH

49.9 g (0.1 mol) of 9,11,15-triacetoxyprost-13-en-1-oic acid was dissolved in 300 ml of chloroform. 20.6 g of N, N'-Dicyclohexylcarbodiimide was added into the reaction mixture. 13.1 g of dimethylaminoethyl mercaptan was added into the reaction mixture. The mixture was stirred for 3 hours at RT. The solid was removed by filtration. The chloroform solution was washed with 5% NaHCO₃ (2 x 100 ml) and water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 45 g of the desired product (85.8%). Hygroscopic product. Solubility in water: 100 mg/ml; Elementary analysis: C₃₂H₅₃NO₉; MW: 657.9 Calculated % C: 64.51; H: 8.97; N: 2.35; O: 24.17; Found % C: 64.47; H: 8.99; N: 2.34, O: 24.20. ¹H-NMR (400 MHz, D₂O): δ: 0.95 (t, 3H), 1.25-1.31 (m, 6H), 1.54 (m, 2H) 1.56 (t, 6H), 1.72 (m, 2H), 1.95 (m, 2H), 2.01 (s, 3H), 2.02 (s, 3H), 2.10 (m, 1H), 2.18 (s, 3H), 2.20 (m, 2H), 2.25 (t, 2H), 2.30 (m, 2H), 3.18 (m, 1H), 3.22 (m, 4H), 3.50 (m, 2H), 4.50 (m, 1H), 4.52 (m, 2H), 4.58 (m, 1H), 5.45-5.69 (m, 4H).

### Preparation of N,N-diethylaminoethyl

### 9,11,15-trihydroxyprosta-5,13-dien-1-oate.AcOH

37.7 g (0.1 mol) of sodium N,N-diethylaminoethyl 9,11,15-trihydroxyprosta-5,13-dien-1-oate was dissolved in 100 ml of acetonitrile. 39 g (0.15 mol) of 2-Bromo-N,N-diethylethylamine.HBr in ethyl acetate was added into the reaction mixture. The mixture was stirred for 3 h at RT. Then 8 g of sodium bicarbonate wass added into the reaction mixture. The mixture is stirred for another 2 h at RT. The solvents were evaporated off. 250 ml of ethyl acetate was added into the reaction mixture and the mixture was washed with water (3 x 100 ml). The organic solution was dried over anhydrous sodium sulfate. Sodium sulfate was removed by filtration. 6 g of acetic acid was added into the reaction mixture with stirring. Hexane (200 ml) was added. The solid product was collected by filtration. After drying, it yielded 45 g of the desired product (87.6%). Hygroscopic product; Solubility in water: 100 mg/ml; Elementary analysis: C₂₈H₅₁NO₇; MW: 513.71. Calculated % C: 65.47; H: 10.01; N: 2.73; O: 21.80; Found % C: 65.42; H: 10.03; N: 2.70; O: 21.85. ¹H-NMR (400 MHz, D₂O): δ: 0.96 (t, 3H), 1.25-1.33 (m, 6H), 1.48 (m, 2H), 1.55 (t, 6H), 1.65 (m, 1H), 1.72 (m, 2H), 1.81 (m, 2 H), 1.92 (m, 2 H), 1.96 (m, 2 H), 2.26 (m, 1H), 2.18 (s, 3H), 2.25 (t, 2H), 3.21 (m, 1H), 3.23 (m, 1H), 3.25 (m, 4H), 3.52 (m, 2 H), 3.86 (m, 1H), 4.52 (m, 2 H), 5.65-5.69 (m, 4H).

### Preparation of N,N-diethylaminoethyl

### 9,11,15-trihydroxy-15-methylprosta-5,13-dien-1-oate.AcOH

60 g of Polymer-bound triethylamine (3 mol/g, 100-200 mesh) was suspended in 180 ml of chloroform. 29.6 g (0.1 mol) of N,N-diethylaminoethyl 9,11,15-trihydroxy-15-methylprosta-5,13-dien-1-oic acid was added into the mixture with stirring. 43 g (0.15mol) of N,N-diethylaminoethyl bromide.HBr was added into the mixture and the mixture was stirred for 5 hours at RT. The polymer was removed by filtration and washed with tetrahydrofuran (3 x 50 ml). 8.2 g (0.1 mol) of sodium acetate was added into the reaction mixture with stirring. The mixture was stirred for 2 h. The solid was removed by filtration and washed with chloroform (3 x 50 ml). The solution was concentrated in vacuo to 100 ml. Then 300 ml of hexane was added into the solution. The solid product was collected by filtration and washed with hexane (3 x 100 ml). After drying, it yielded 47 g of the desired product (87.8%). Hygroscopic product; Solubility in water: 100 mg/ml; Elementary analysis: C₂₈H₅₁NO₇; MW: 527.73. Calculated % C: 66.00; H: 10.12; N: 2.65; O: 21.22; Found % C: 65.96; H: 10.15; N: 2.64; O: 21.24. ¹H-NMR (400 MHz, D₂O): δ: 0.95 (t, 3H), 1.24-1.34 (m, 6H), 1.41 (s, 3 H), 1.47 (m, 2H), 1.56 (t, 6H), 1.65 (m, 1H), 1.72 (m, 2H), 1.82 (m, 2 H), 1.92 (m, 2 H), 1.97 (m, 2 H), 2.26 (m, 1H), 2.18 (s, 3H), 2.25 (t, 2H), 3.21 (m, 1H), 3.23 (m, 1H), 3.25 (m, 4H), 3.52 (m, 2 H), 4.52 (m, 2 H), 5.64-5.68 (m, 4H).

### Industrial Applicability

The pro-drugs of the general formula (2) 'Structure 2' are superior to prostaglandins, prostacyclins and related compounds. They can be used medicinally in treating any prostaglandins, prostacyclins and related compounds-treatable conditions in humans or animals. They may be used for the treatment of glaucoma or ocular hypertension, treatment of male erectile dysfunction and to enhance female sexual arousal, reducing systemic blood pressure, abortion, hypotensive control, inhibition of platelet aggregation, treatment of pulmonary diseases, gastrointestinal disease, shock, reproduction, fertility, etc.

### Sequence List Text

### Further Embodiments

1. The compounds of the general formula (2) 'Structure 2' 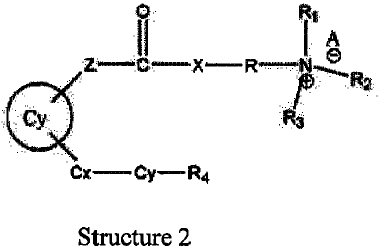 Wherein, R₁ represents H, one of any alkyl, alkyloxyl, alkenyl or alkynyl residues having 1 to 12 carbon atoms, aryl or heteroaryl residues; R₂ represents H, one of any alkyl, alkyloxy, alkenyl or alkynyl residues having 1 to 12 carbon atoms, aryl or heteroaryl residues; R₃ represents H, one of any alkyl, alkyloxy, alkenyl or alkynyl residues having 1 to 12 carbon atoms, aryl or heteroaryl residues; X represents O, S, or NH; A- represents Cl⁻, Br⁻, F⁻, I⁻, AcO⁻, citrate, or any negative ions; R represents a branched or straight chain, -(CH₂)ₙ-, wherein n=0, 1, 2, 3, 4, 5, 6, 7, 8, 9, 10 ......, an aryl or heteroaryl group; Cx-Cy is -CH₂-CH₂-, -S-CH₂-, -O-CH₂-, -C≡C-, or -CH=CH-; Z represents -(CH₂)₆-, -(CH₂)ₙ-, -(CH₂)ₘ-O-CH₂-, -(CH₂)ₘ-S-CH₂-, -CH₂C≡C-(CH₂)ₙ-, -CH₂C=C-(CH₂)ₙ-O-CH₂-, -CH₂C≡C-(CH₂)ₙ-S-CH₂-, -CH₂-CO-(CH₂)ₙ-, -CH₂-CH=C=CH-(CH₂)ₙ-, -CH₂-CH=C=CH-O-(CH₂)ₙ-, -CH₂-CH=C=CH-S-(CH₂)ₙ-, 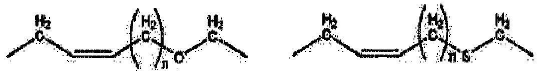 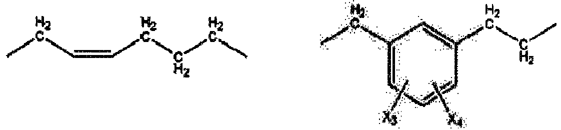 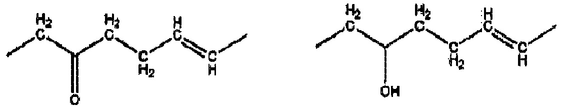 Wherein m and n have a value of 0 to 6 inclusive; R₄ represents: 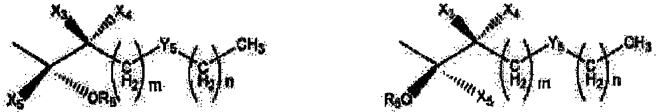 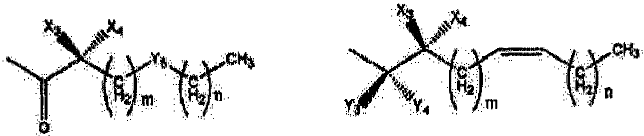 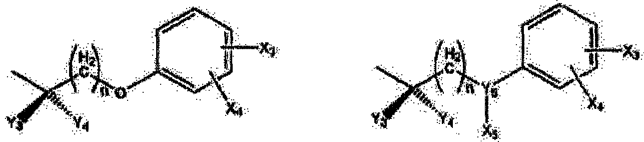 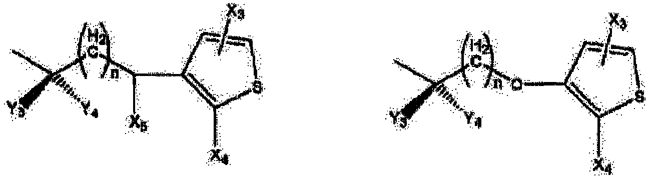 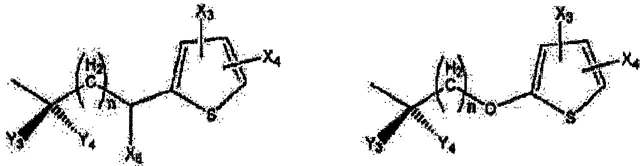 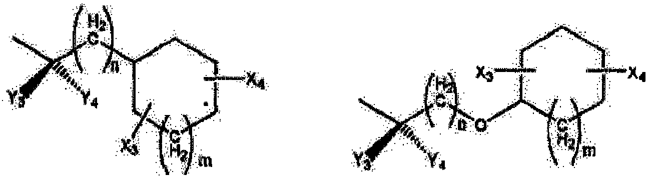 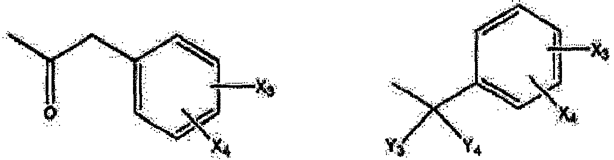 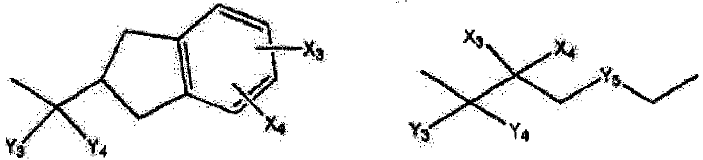 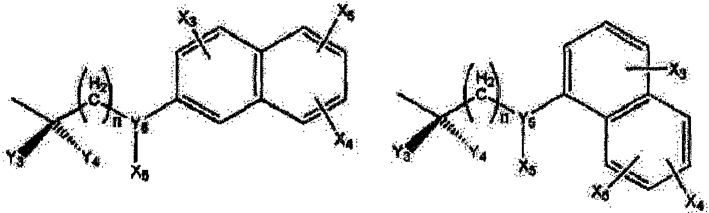 Wherein R₅ represents H, OH, acetyl, propionyl, isobutyryl, butyryl, pivaloyl, valeryl, and isovaleryl; X₃, X₄, and X₅ represent H, OH, Cl, F, OCH₃, S-CH₃, CH₃, C₂H₅, CH=CH₂, CH₂CH=CH₂, and CF₃; Y₃ and Y₄ taken alone are different and are H, OH, OR₅, OOH, OCOCH₃, OCOC₂H₅, OCOC₃H₇, OCOC₄H₉, OCOC₅H₁₁, OCOC₆H₁₃, CH₃, CH₂OH, CH₂OCOCH₃, CH₂OCOC₂H₅, CH₂OCOC₃H₇, CH₂OCOC₄H₉, Cl, F, Br, I, or taken together are oxygen or 2 hydrogens. Y₅ is CH₂, NH, S, or O, m and n have a value of 0 to 8 inclusive. 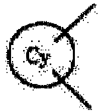 represents; 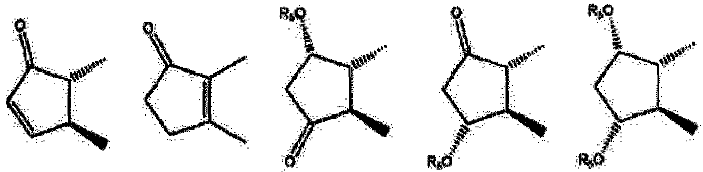 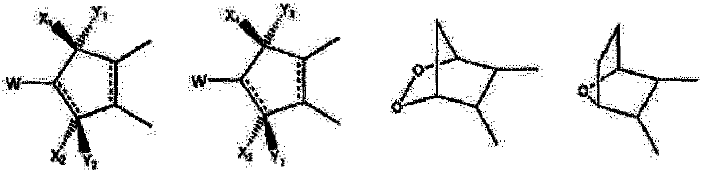 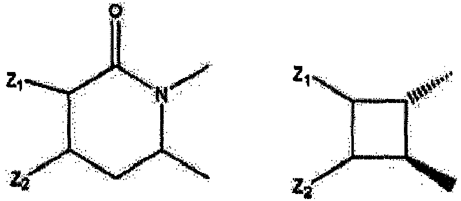 Wherein R₅ represents H, OH, acetyl, propionyl, isobutyryl, butyryl, pivaloyl, valeryl, and isovaleryl; X₁ and Y₁ taken alone are different and are H, OH, OR₅, OOH, OCOCH₃, OCOC₂H₅, OCOC₃H₇, OCOC₄H₉, OCOC₅H₁₁, OCOC₆H₁₃, CH₂-OH, Cl, F, Br, I, or taken together are oxygen or 2 hydrogens; X₂ and Y₂ taken alone are different and are H, OH, OOH, OCOCH₃, OCOC₂H₅, OCOC₃H₇, OCOC₄H₉, OCOC₅H₁₁, OCOC₆H₁₃, CH₂-OH, Cl, F, Br, I, nothing (when the dashed bond is a double bond) or taken together are oxygen or 2 hydrogens; Z₁ and Z₂ represent H, OH, OR₅, OOH, OCOCH₃, OCOC₂H₅, OCOC₃H₇, OCOC₄H₉, OCOC₅H₁₁, OCOC₆H₁₃, CH₂-OH, or Cl. W represents H, CH , Cl, F, Br, I, or OH; the dashed bonds represent a single or double bond; All R, -(CH₂)ₙ- or -(CH₂)ₘ-groups are branched or straight chains and may include C, H, O, S, or N atoms and may have single, double, and triple bonds. Any CH₂ groups may be replaced with O, S, or NH.
2. Processes for the preparation of compounds of the general formula (2) 'Structure 2' according to Embodiment 1, wherein the compounds can be prepared from protected prostaglandins, prostacyclins, and related compounds , by reaction with compounds of the general formula (3) 'Structure 3' by using coupling reagents, such as N,N'-Dicyclohexylcarbodiimide, N, N'-Diisopropylcarbodiimide, O- (Benzotriazol-1-yl)-N,N,N',N'-tetramethyluronium tetrafluoroborate, O- (Benzotriazol-1-yl)-N,N,N',N'-tetramethyluronium hexafluorophosphate, Benzotriazol-1-yl-oxy-tris(dimethylamino)phosphonium hexafluorophosphate, et al. 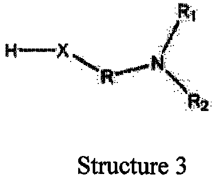 Wherein, R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R represents a branched or straight chain, - (CH₂)ₙ-, wherein n=0, 1, 2, 3, 4, 5, 6, 7, 8, 9, 10 ......, aryl or heteroaryl groups; X represents O, S or NH; and n=0, 1, 2, 3, 4, 5, 6, 7, 8, 9, 10......
3. Processes for the preparation of compounds of the general formula (2) 'Structure 2' according to Embodiment 1, wherein the compounds can be prepared from metal salts, organic base salts, or immobilized base salts of prostaglandins, prostacyclins and related compounds, by reaction with compounds of the general formula (4) 'Structure 4'. 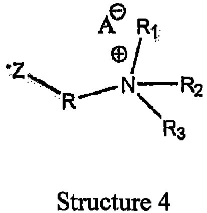 Wherein, R₁ represents H, one of any alkyl, alkyloxy, alkenyl, or alkynyl residues having 1 to 12 carbon atoms, aryl or heteroaryl residues; R₂ represents H, one of any alkyl, alkyloxy, alkenyl, or alkynyl residues having 1 to 12 carbon atoms, aryl or heteroaryl residues; R₃ represents H, one of any alkyl, alkyloxy, alkenyl, or alkynyl residues having 1 to 12 carbon atoms, aryl or heteroaryl residues; R represents a branched or straight chain, -(CH₂)ₙ -, wherein n=0, 1, 2, 3, 4, 5, 6, 7, 8, 9, 10 ......, aryl or heteroaryl groups; Z represents halogen, or p- toluenesulphonyl, A⁻ represents Cl⁻, Br⁻, F⁻, I⁻, AcO⁻, citrate, or any negative ions; and n=0, 1, 2, 3, 4, 5, 6, 7, 8, 9, 10.....
4. Compounds of the general formula (2)'Structure 2' or a composition comprising of at least one compound of the general formula (2) 'Structure 2', as an active ingredient, according to Embodiment 1, can be administered orally or transdermally, for treating any prostaglandins, prostacyclins and related compounds-treatable conditions in humans or animals. The prostaglandins, prostacyclins and related compounds-treatable conditions include, but are not limited to glaucoma or ocular hypertension, male erectile dysfunction and female sex dysfunction, systemic high blood pressure, abortion, hypotensive control, inhibition of platelet aggregation, pulmonary diseases, gastrointestinal disease, inflammation, shock, reproduction, fertility, etc,.
5. Methods for treating any prostaglandins, prostacyclins and related compounds- treatable conditions in humans or animals by administering transdermally to any part of body (in the form of a solution, spray, lotion, ointment, emulsion or gel) to deliver therapeutically effective plasma levels of the compounds of the general formula (2) 'Structure 2' or a composition comprising of at least one compound of the general formula (2) 'Structure 2', as an active ingredient, according to Embodiment 1.
6. Methods for the treatment of a male erectile dysfunction or female sex dysfunction by transdermally administering (in the form of a solution, spray, lotion, ointment, emulsion or gel) to the genital area of a male or female human or animal a therapeutically effective amount of the compounds of the general formula (2) 'Structure 2' or a composition comprising of at least one compound of the general formula (2) 'Structure 2', as an active ingredient, according to Embodiment 1.
7. Methods for the treatment of glaucoma or ocular hypertension by transdermally administering (in the from of a solution, spray, lotion, ointment, emulsion) to the eye of a human or animal a therapeutically effective amount of the compounds of the general formula (2) 'Structure 2' or a composition comprising of at least one compound of the general formula (2) 'Structure 2', as an active ingredient, according to Embodiment 1.
8. Methods for the treatment of systemic high blood pressure by transdermally administering (in the form of a solution, spray, lotion, ointment, emulsion or gel) to any part of a human or animal a therapeutically effective amount of the compounds of the general formula (2) 'Structure 2' or a composition comprising of at least one compound of the general formula (2) 'Structure 2', as an active ingredient, according to Embodiment 1.
9. Compounds of the general formula (2) 'Structure 2' or a composition comprising of at least one compound of the general formula (2) 'Structure 2', as an active ingredient, according to Embodiment 1, can be administered transdermally (in the form of a solution, spray, lotion, ointment, emulsion or gel) to the vagina of a female, as an abortifacient.
10. Methods for the treatment of gastrointestinal ulcers and ulcers of the skin by transdermally administering (in the form of a solution, spray, lotion, ointment, emulsion or gel) to any part of a human or animal a therapeutically effective amount of the compounds of the general formula (2) 'Structure 2' or a composition comprising of at least one compound of the general formula (2) 'Structure 2', as an active ingredient, according to Embodiment 1.
11. Methods for the treatment of inflammation by transdermally admnistering to the eye or other body part of a human or animal a therapeutically effective amount of the compounds of the general formula (2) 'Structure 2' or a composition comprising of at least one compounds of the general formula (2) 'Structure 2', as an active ingredient, according to Embodiment 1.
12. Transdermal therapeutic application systems of compounds of the general formula (2) 'Structure 2' or a composition comprising of at least one compound of the general formula (2) 'Structure 2', as an active ingredient, according to Embodiment 1, for treating any prostaglandins, prostacyclins and related compounds-treatable conditions in humans or animals. These systems can be a bandage or a patch comprising of one active substance-containing matrix layer and an impermeable backing layer. The most preferable system is an active substance reservoir, which has a permeable bottom facing the skin. By controlling the rate of release, this system enables the prostaglandins, prostacyclins and related compounds to reach constantly optimal therapeutic blood levels to increase effectiveness and reduce the side effects of prostaglandins, prostacyclins and related compounds.

## Claims

1. A compound having a general formula of Structure 2 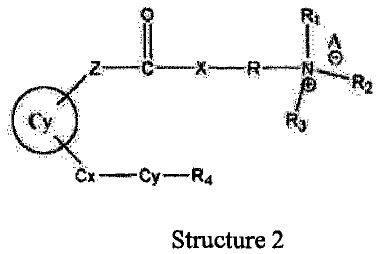 wherein
R₁ is selected from the group consisting of H, alkyl, alkyloxyl, alkenyl, and alkynyl residues having 1 to 12 carbon atoms, aryl, and heteroaryl residues;
R₂ is selected from the group consisting of H, alkyl, alkyloxy, alkenyl, and alkynyl residues having 1 to 12 carbon atoms, aryl, and heteroaryl residues;
R₃ represents H;
X represents O, S, and NH;
A⁻ is selected from any negative ions;
R represents a branched or straight chain, -(CH₂)ₙ-, wherein n=0, 1, 2, 3, 4, 5, 6, 7, 8, 9, or 10....., an aryl group, and a heteroaryl group; Cx-Cy is-CH₂- CH₂-, -S-CH₂-, -O-CH₂-, -C≡C-, and -CH=CH-;
Z represents -(CH₂)₆-, -(CH₂)ₙ-, -(CH₂)ₘ-O-CH₂-, -(CH₂)ₘ-S-CH₂-, -CH₂C≡C-(CH₂)ₙ-, - CH₂C=C-(CH₂)ₙ-O-CH₂-, -CH₂C≡C-(CH₂)ₙ-S-CH₂-, -CH₂-CO-(CH₂)ₙ-, -CH₂-CH=C=CH-(CH₂)ₙ-, -CH₂-CH=C=CH-O-(CH₂)ₙ-, -CH₂-CH=C=CH-S-(CH₂)ₙ-, 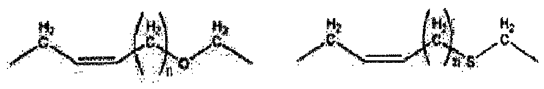 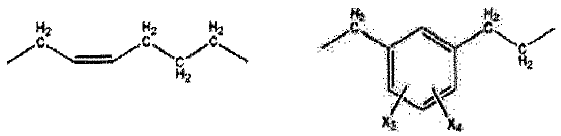 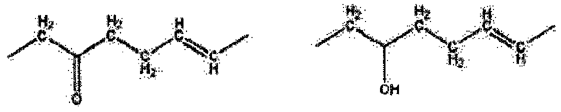
wherein m and n have a value of 0 to 6 inclusive; R₄ represents: 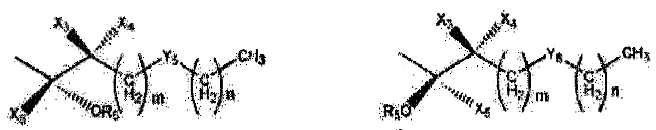 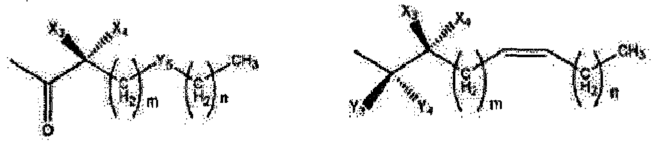 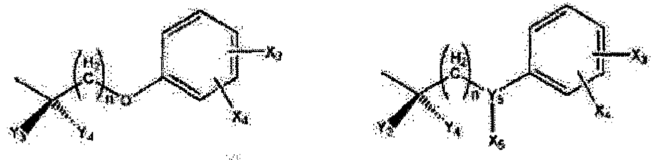 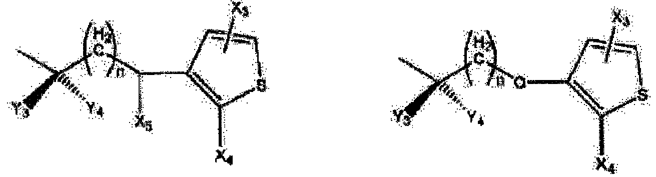 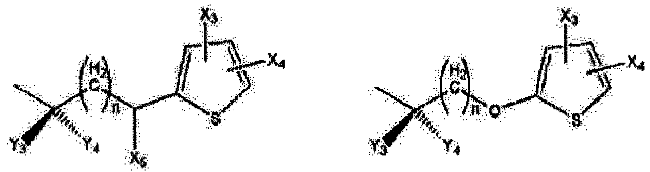 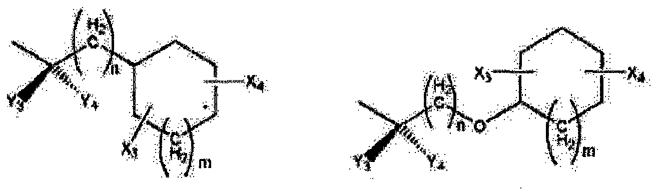 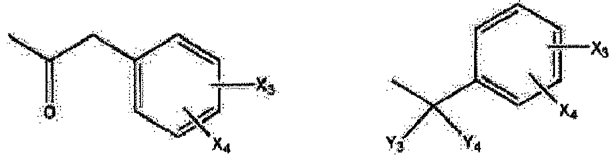 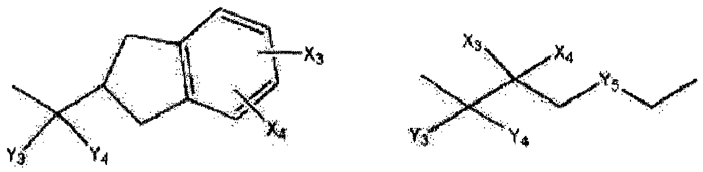 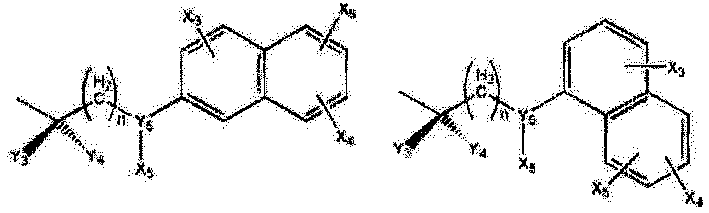
wherein R₅ represents H, OH, acetyl, propionyl, isobutyryl, butyryl, pivaloyl, valeryl, and isovaleryl;
X₃, X₄, and X₅ represent H, OH, Cl, F, OCH₃, S-CH₃, CH₃, C₂H₅, CH=CH₂, CH₂CH=CH₂, and CF₃;
Y₃ and Y₄ taken alone are different and are H, OH, OR₅, OOH, OCOCH₃, OCOC₂H₅, OCOC₃H₇, OCOC₄H₉, OCOC₅H₁₁, OCOC₆H₁₃, CH₃, CH₂OH, CH₂OCOCH₃, CH₂OCOC₂H₅, CH₂OCOC₃H₇, CH₂OCOC₄H₉, Cl, F, Br, and I, or taken together are oxygen or 2 hydrogens;
Y₅ is CH₂, NH, S, and O, m and n have a value of 0 to 8 inclusive 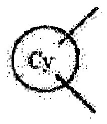 represents: 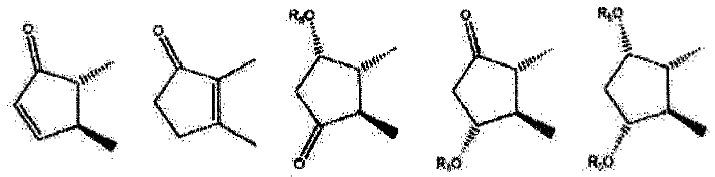 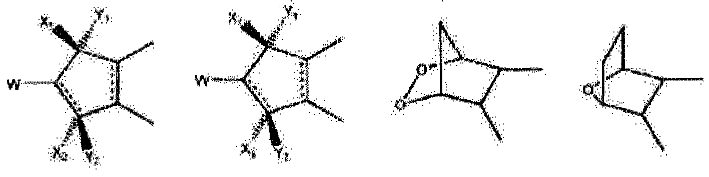 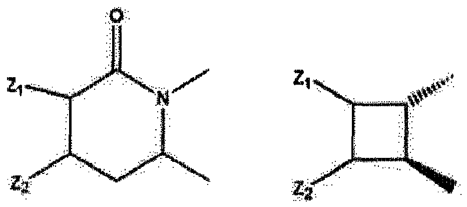
wherein R₅ represents H, OH, acetyl, propionyl, isobutyryl, butyryl, pivaloyl, valeryl, and isovaleryl;
X₁ and Y₁ taken alone are different and are H, OH, OR₅, OOH, OCOCH₃, OCOC₂H₅, OCOC₃H₇, OCOC₄H₉, OCOC₅H₁₁, OCOC₆H₁₃, CH₂-OH, Cl, F, Br, and I, or taken together are oxygen or 2 hydrogens;
X₂ and Y₂ taken alone are different and are H, OH, OOH, OCOCH₃, OCOC₂H₅, OCOC₃H₇, OCOC₄H₉, OCOC₅H₁₁, OCOC₆H₁₃, CH₂-OH, Cl, F, Br, I, and nothing (when the dashed bond is a double bond) or taken together are oxygen or 2 hydrogens;
Z₁ and Z₂ represent H, OH, OR₅, OOH, OCOCH₃, OCOC₂H₅, OCOC₃H₇, OCOC₄H₉, OCOC₅H₁₁, OCOC₆H₁₃, CH₂-OH, and Cl;
W represents H, CH , Cl, F, Br, I, and OH;
the dashed bonds represent a single or double bond;
all R, -(CH₂)ₙ- or -(CH₂)ₘ- groups are branched or straight chains and may include C, H, O, S, or N atoms and may have single, double, and triple bonds; and
any CH₂ groups may be replaced with O, S, or NH.

2. The compound according to claim 1, wherein A⁻ is selected from the group consisting of Cl⁻, Br⁻, F⁻, I⁻, and AcO⁻.

3. A composition comprising one or more compounds according to claim 1.

4. The composition according to claim 3, further comprising water.

5. The compound according to claim 1 or 2, or the composition according to claim 3 or 4, for use in a method of treatment in human or animal, wherein the treated condition is treating a condition treatable by a prostaglandin, a prostacyclin, and/or a related compound.

6. The compound or composition for use according to 5, wherein the treated condition is selected from the group consisting of glaucoma, ocular hypertension, male erectile dysfunction, female sex dysfunction, systemic high blood pressure, abortion, hypotensive control, inhibition of platelet aggregation, pulmonary diseases, gastrointestinal disease, gastrointestinal ulcers, skin ulcers, inflammation, eye inflammation, shock, reproduction, and fertility.

7. The compound or composition for use according to claim 5 or 6, wherein the compound or composition is administered to deliver a therapeutically effective plasma level of the compounds or the composition.

8. The compound or composition for use according to any of claims 5 to 7, wherein the compound or composition is administered in the form of a solution, spray, lotion, ointment, emulsion or gel.

9. The compound or composition for use according to any of claims 5 to 8, wherein the compound or composition is administered orally or transdermally.

10. The compound or composition for use according to any of claims 5 to 8, wherein the compound or composition is administered to a body part of the human or animal selected from the group consisting of a genital area of a male, a genital area of a female, an eye, and a vagina of a female.

11. Transdermal therapeutic application systems comprising a compounds according to claim 1 or 2, or a composition according to claim 3 or 4.

12. The transdermal therapeutic application systems according to claim 11, **characterized in that** the system is a bandage or a patch comprising an active-substance-containing matrix layer, and an impermeable backing layer.

13. The transdermal therapeutic application systems according to claim 11 or 12, **characterized by** having an active substance reservoir, which has a permeable bottom facing the skin.

14. The transdermal therapeutic application systems according to any of claims 11 to 13, **characterized by** a controlling means for controlling the rate of release, enabling the system to reach constantly optimal therapeutic blood levels.

15. The transdermal therapeutic application systems according to any of claims 11 to 14, for use in a method of treatment in human or animal, wherein the treated condition is treating a condition treatable by a prostaglandin, a prostacyclin, and/or a related compound.
